# EUROPEAN PATENT SPECIFICATION

(11) **EP 4 329 868 B1**
(45) Date of publication and mention of the grant of the patent: **12.11.2025**
(21) Application number: 21737212.7
(22) Date of filing: 19.05.2021
(51) Int. Cl.: A61M 39/02, A61M 25/00, A61M 25/01, A61M 39/12

(54) **CATHLOCK COUPLING TOOL**
KATHETERKUPPLUNGSWERKZEUG
OUTIL DE COUPLAGE À VERROU DE CATHÉTER

(43) Date of publication of application: 06.03.2024
(73) Proprietor: Bard Peripheral Vascular, Inc., Franklin Lakes, NJ 07417 (US)
(72) Inventor: ANDERSEN, Christian, Kaysville, UT 84037 (US); FIUMEFREDDO, Diana, Salt Lake City, UT 84121 (US); THOMAS, Ian, N., Bountiful, UT 84087 (US); HOYE, Jessica, Phoenix, AZ 85032 (US); DENSLEY, Bryon, Ray, Fremont, CA 94538 (US)
(74) Representative: Hoffmann Eitle
(86) International application number: PCT/US2021/033206
(87) International publication number: WO 2022/245350

(56) References cited:
- WO-A1-2021/041367
- US-A- 5 129 891

## Description

### BACKGROUND

WO 2021/041367 A1 discloses a fluid delivery device, system and method including a subcutaneous port, a catheter, and a connection assembly configured to fluidly couple the port and catheter.

US 5129891 A discloses a connector for detachably securing an end of a tube, such as a catheter, to a fluid port of a fluid transfer assembly, such as an implantable device.

### SUMMARY

Briefly summarized, embodiments disclosed herein are directed to a tool for coupling a cathlock to a port to secure a catheter to a port stem.

Proximally trimmable catheters allow for post-placement sizing of the catheter. When placing a catheter and port assembly, the position of the distal tip of the catheter can be important for the efficacy of the treatment. For example, when placing a catheter within the superior vena cava, if the distal tip of the catheter falls short of the target area, the efficacy of the medicament is reduced. If the distal tip is advanced too far, the distal tip can cause arrhythmia. The distance between the distal tip of the catheter and the port can vary since the distances between the target location, insertion site to the vasculature, and the location of the port can vary between patients and procedures. Estimating the catheter length before placement can lead to errors that result in misplacement of the distal tip.

Proximally trimmable catheters allow for placement of the catheter distal tip at the target location before trimming a proximal portion of the catheter to the correct length. The clinician can then attach the catheter to a subcutaneous port, or similar access device. However, securing the catheter to the port can be challenging. The connection must be leak-proof, especially under high-pressure infusion. Further, manipulating the catheter and port within the confined, wetted environment of a subcutaneous access site can lead to slippage, undue trauma to the access site, or misplacement of the catheter distal tip.

Disclosed herein is a system for coupling a cathlock to a port stem to secure a catheter thereto including, a cathlock defining a lumen extending longitudinally from a first end to a second end and a tool including a body defining a channel extending along a longitudinal axis from a first end to a second end, the channel including a first opening at the first end and configured to receive the cathlock therein, the channel further including a second opening at the second end, and a handle extending from the body at an angle relative to the longitudinal axis, the handle coupled to one of the first end or the second end of the body. The body further includes an elongate opening defining a first edge and a second edge, the elongate opening extending longitudinally between the first end and the second end, and communicating with the channel. The body is formed of a resilient material and elastically deformable

In some embodiments, the handle is coupled to the first end of the body and the body extends from the handle to the second end. In some embodiments, the handle is coupled to the second end of the body and the body extends from the handle to the first end. In some embodiments, the second edge is disposed adjacent to the handle. In some embodiments, the first edge contacts the second edge when the body is in an unstressed state to form a slit. In some embodiments, the elongate opening extends through an arc distance between the first edge and the second edge, the arc distance being between 0° and 90°.

In some embodiments, the channel defines a cylindrical shape profile. In some embodiments, a wall of the channel extends parallel to the longitudinal axis. In some embodiments, the channel defines a tapered profile. In some embodiments, a wall of the channel extends at an angle relative to the longitudinal axis. In some embodiments, a diameter of the first opening is larger than a diameter of the second opening. In some embodiments, the first opening is larger than a diameter of the second end of the cathlock, and the second opening is smaller than the diameter of the second end of the cathlock.

In some embodiments, the channel defines a continuous decrease in diameter between the first opening and the second opening. In some embodiments, the channel defines a discontinuous decrease in diameter between the first opening and the second opening. In some embodiments, the channel includes an abutment configured to abut against a surface of the cathlock and to inhibit further axial movement. In some embodiments, the inner profile of the channel mirrors an outer profile of the cathlock.

Also disclosed is a method of coupling a cathlock to a port stem to secure a catheter thereto including, slidably engaging a cathlock with a catheter, the cathlock defining a cathlock lumen extending longitudinally from a first end to a second end, urging a port stem into a lumen of the catheter, coupling a body of a tool with the cathlock, the body defining a channel extending longitudinally along an axis from a first opening to a second opening, and including a handle extending from the body at an angle relative to the axis of the channel, the handle coupled with the body adjacent one of the first opening or the second opening, and urging the tool axially towards the port stem to couple the cathlock thereto, compressing a portion of the catheter onto the port stem.

In some embodiments, the handle is coupled to a first end of the body, the body extending from the handle to a second end of the body. In some embodiments, the handle is coupled to a second end of the body, the body extending from the handle to a first end of the body. In some embodiments, the method further includes urging one of a portion of the catheter or the cathlock through an elongate opening disposed in the body and communicating with the channel, the elongate opening extending longitudinally and defining a first edge and a second edge. In some embodiments, the second edge is disposed adjacent to the handle. In some embodiments, the first edge contacts the second edge when the body is in an unstressed state to form a slit. In some embodiments, the elongate opening extends through an arc distance between the first edge and the second edge, the arc distance being between 0° and 90°.

In some embodiments, the channel defines a cylindrical shape profile. In some embodiments, a wall of the channel extends parallel to the longitudinal axis. In some embodiments, the channel defines a tapered profile. In some embodiments, a wall of the channel extends at an angle relative to the longitudinal axis. In some embodiments, a diameter of the first opening of the channel is larger than a diameter of the second opening of the channel. In some embodiments, the first opening of the channel is larger than a diameter of the second end of the cathlock and the second opening of the channel is smaller than the diameter of the second end of the cathlock.

In some embodiments, the channel defines a continuous decrease in diameter between the first opening and the second opening. In some embodiments, the channel defines a discontinuous decrease in diameter between the first opening and the second opening. In some embodiments, the channel includes an abutment configured to abut against a surface of the cathlock and to inhibit further axial movement. In some embodiments, the inner profile of the channel mirrors an outer profile of the cathlock.

Also disclosed is a kit for accessing a vasculature of a patient including, a port having a port stem, a catheter defining a catheter lumen configured to engage the port stem, a cathlock defining a cathlock lumen extending from a first end to a second end and configured to slidably engage the catheter, the cathlock configured to engage an outer surface of the catheter and to secure the catheter to the port stem, and a cathlock tool configured to engage a surface of the cathlock, the cathlock tool having, a body extending longitudinally from a first end to a second end and defining a channel communicating with a first opening disposed at the first end, the first opening configured to receive the cathlock therethrough, and a handle extending from the body at an angle relative to an axis of the channel and coupled to one of the first end or the second end of the body.

In some embodiments, the port includes a port body defining a reservoir in fluid communication with a lumen of the port stem. In some embodiments, the port includes a septum disposed over the reservoir and configured to provide percutaneous access thereto. In some embodiments, the cathlock is configured to engage one of the port or the port stem in an interference fit, press-fit, or snap-fit engagement. In some embodiments, the cathlock is configured to compress a portion of the catheter onto the port stem. In some embodiments, an inner profile of the channel of the tool mirrors an outer profile of the cathlock. In some embodiments, the channel defines one of a cylindrical or tapered outer profile. In some embodiments, the channel includes an abutment surface configured to abut against a surface of the cathlock and prevent further axial movement of the cathlock through the channel.

In some embodiments, the body of the tool further includes an elongate opening communicating with the channel and configured to provide ingress or egress to of one of the cathlock or the catheter to or from the channel. In some embodiments, the handle is coupled to the first end of the body and the body extends from the handle to the second end. In some embodiments, the handle is coupled to the second end of the body and the body extends from the handle to the first end. In some embodiments, the channel further includes a second opening disposed at the second end of the body, a diameter of the first opening being larger than a diameter of the second opening. In some embodiments, the first opening is larger than a diameter of the second end of the cathlock, and the second opening is smaller than the diameter of the second end of the cathlock.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a perspective view of a cathlock coupling tool in an exemplary environment of use, in accordance with embodiments disclosed herein.
FIG. 2A illustrates a perspective view of a cathlock coupling tool, in accordance with embodiments disclosed herein.
FIG. 2B illustrates a perspective axial view of the cathlock coupling tool, in accordance with embodiments disclosed herein.
FIG. 3A illustrates a side view of a cathlock coupling tool, in accordance with embodiments disclosed herein.
FIG. 3B illustrates a side view of an embodiment of a cathlock coupling tool, in accordance with embodiments disclosed herein.
FIGS. 4A-4E illustrate an exemplary method of use for a cathlock coupling tool, in accordance with embodiments disclosed herein.
FIG. 5 illustrates a flow chart of an exemplary method of use for the cathlock coupling tool, in accordance with embodiments disclosed herein.
FIG. 6A shows a cross-sectional view of a cathlock, in accordance with embodiments disclosed herein.
FIG. 6B shows a side view of a cathlock, in accordance with embodiments disclosed herein.
FIG. 6C shows a cross-sectional view of a cathlock, in accordance with embodiments disclosed herein.
FIG. 6D shows a side view of a cathlock, in accordance with embodiments disclosed herein.
FIGS. 6E-6F show perspective views of a cathlock, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal-end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal-end portion, or the proximal length of the catheter can include the proximal end of the catheter; however, the proximal portion, the proximal-end portion, or the proximal length of the catheter need not include the proximal end of the catheter. That is, unless context suggests otherwise, the proximal portion, the proximal-end portion, or the proximal length of the catheter is not a terminal portion or terminal length of the catheter.

With respect to "distal," a "distal portion" or a "distal-end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal-end portion, or the distal length of the catheter can include the distal end of the catheter; however, the distal portion, the distal-end portion, or the distal length of the catheter need not include the distal end of the catheter. That is, unless context suggests otherwise, the distal portion, the distal-end portion, or the distal length of the catheter is not a terminal portion or terminal length of the catheter.

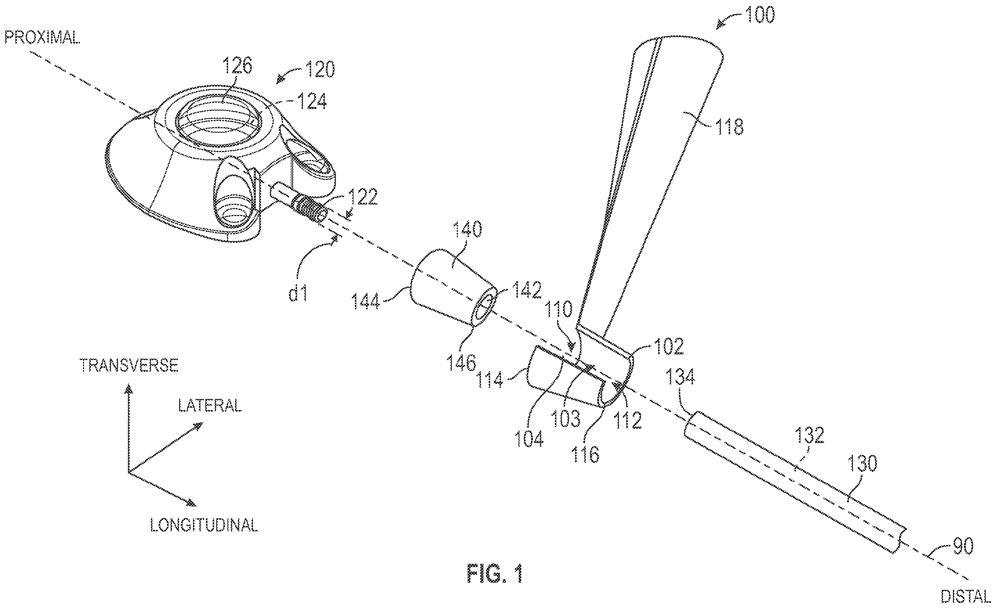

To assist in the description of embodiments described herein, as shown in FIG. 1, a longitudinal axis extends substantially parallel to an axial length of a channel 103 of the tool body 102. A lateral axis extends normal to the longitudinal axis, and a transverse axis extends normal to both the longitudinal and lateral axes.

Unless defined otherwise, all technical and scientific terms used herein have the same meaning as commonly understood by those of ordinary skill in the art.

FIG. 1 shows a perspective view of a cathlock coupling tool ("tool") 100 in an exemplary environment of use. In an embodiment, the tool 100 can be used to engage a cathlock 140 and facilitate coupling the cathlock 140 with a stem 122 of a port 120 to secure a catheter 130 thereto. In an embodiment, the port 120 can generally include a reservoir 124 covered by a needle-penetrable septum 126. The reservoir 124 can be in fluid communication with a lumen of the port stem 122 and can be accessed percutaneously by an access needle. As will be appreciated the port 120 is an exemplary medical device and embodiments disclosed herein can be used with various other subcutaneous or supra-cutaneous medical devices such as catheters, extension legs, medical lines, tube connectors, or the like, without limitation. In an embodiment, the port stem 122 can extend longitudinally along a central axis 90 and can define an outer diameter (*d1*) of the port stem 122.

In an embodiment, a catheter 130 can extend longitudinally and define a catheter lumen 132. A distal tip of the catheter 130 can be disposed within a vasculature of the patient to provide fluid communication therewith. A proximal end, i.e. first end 134, of the catheter 130 can be configured to engage the port stem 122 in an interference fit engagement. In an embodiment, an inner diameter (*d2*) of the catheter lumen can be the same as, or less than, an outer diameter (*d1*) of the port stem 122. As such, the first end 134 of the catheter 130 can be urged in a first direction (i.e. a proximal direction) and stretched over the port stem 122 to provide fluid communication between the lumen of the port stem 122 and the catheter lumen 134.

In an embodiment, a cathlock 140 can be configured to engage an outer surface of the catheter 130, adjacent the first end 134, to compress the catheter 130 onto the port stem 122 and provide fluid tight seal therebetween. The cathlock 140 can define a substantially cylindrical or tapered outer profile and can define a cathlock lumen 142 extending longitudinally from a first end 144, proximate the port 120, to a second end 146, proximate the catheter 130. In use, the cathlock 140 can slidably engage an outer surface of the catheter 130 prior to the catheter 130 engaging the port stem 122. Once the first end 134 of the catheter 130 has engaged the port stem 122, the cathlock 140 can be slid axially over the catheter 130 and engage an outer surface of the catheter 130 adjacent the first end 134. The cathlock 140 can compress the catheter 130 onto the port stem 122 to further secure the catheter 130 thereto.

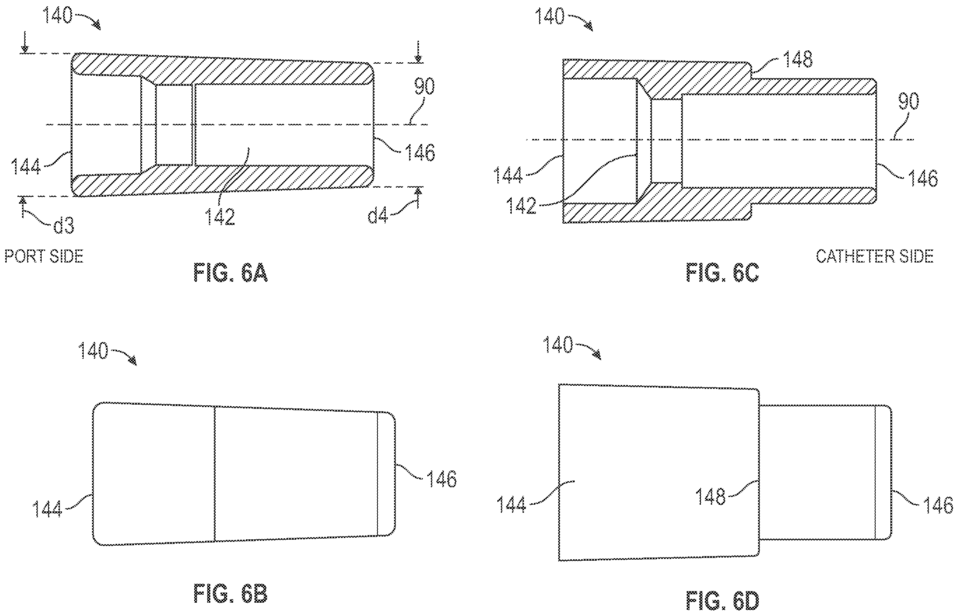

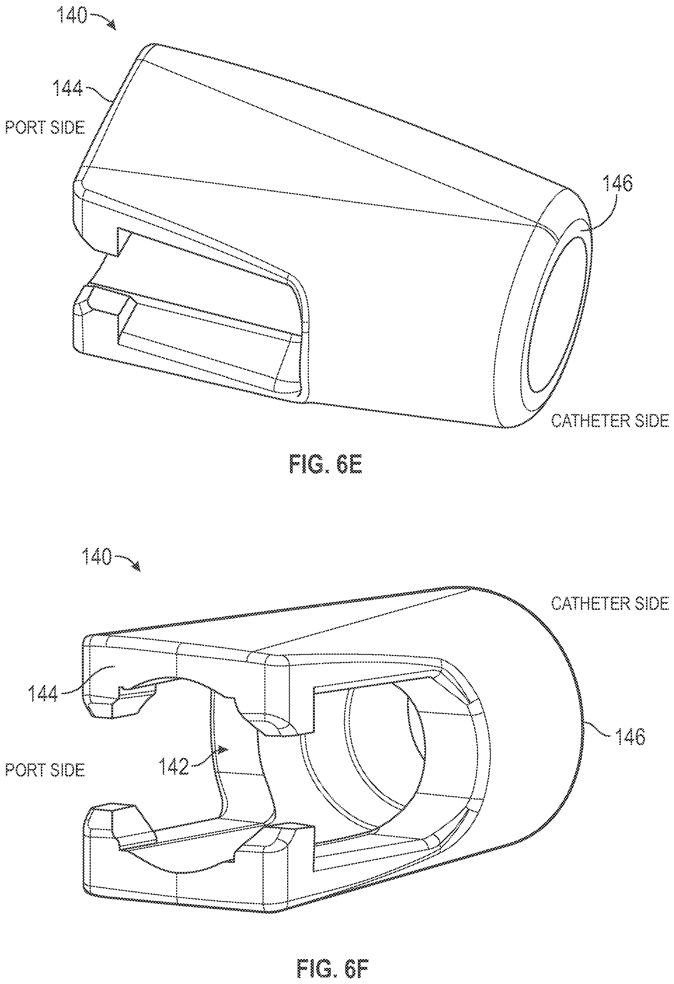

In an embodiment, the cathlock 140 can define various cross-sectional shapes, outer profiles, or sizes. FIGS. 6A-6F gives some exemplary, non-limiting examples of a cathlock 140. In an embodiment, the cathlock 140 can define a continuous outer profile, extending from the first end 144 to the second end 146. The outer profile can define a substantially cylindrical shape and include an outer wall extending substantially parallel to the longitudinal axis. In an embodiment, as shown in FIGS. 6A-6B, the outer profile of the cathlock 140 can define a tapered outer profile, where a portion of an outer surface of the cathlock extends longitudinally at an angle relative to the longitudinal axis. In an embodiment, an outer diameter (d3) of the first end 144 is greater than an outer diameter (d4) of the second end 146 of the cathlock 146. Further, as shown in FIGS. 6A-6B, the outer profile of the cathlock 140 can define a continuous tapered outer profile where the outer profile defines a continuous change in diameter between the first end 144 and the second end 146.

In an embodiment, as shown in FIGS. 6C-6D, the outer profile of the cathlock 140 can define a discontinuous change in diameter between the first end 144 and a second end 146. As such, the outer profile of the cathlock 140 can define one or more straight (cylindrical) portion, a tapered portion extending at one or more angles relative to the longitudinal axis, a stepped portion 148, or combinations thereof.

In an embodiment, as shown in FIGS. 6E-6F, the cathlock 140 can define a circular, elliptical, rectangular cross-sectional shape, or combinations thereof. However, it will be appreciated that other cross-sectional shapes are also contemplated without limitation. In an embodiment, a portion of the cathlock 140 (e.g. a second end 146) can be configured to fit within the channel 103 while the opposite end (e.g. the first end 144) can extend from the body 102, when the cathlock 140 is retained by the tool 100. For example, as shown in FIGS. 6E-6F, a portion of the cathlock 140 adjacent the second end 146, can define a tapered outer profile and configured to fit within the channel 103. As such, an opposite portion of the cathlock 140, adjacent the first end 144 can extend from the channel 103 towards the port 120 and can define a different outer profile. As shown, the opposite portion of the cathlock 140 can define a substantially rectangular shape, or can include one or more engagement structures configured to engage the port 120. In an embodiment, the cathlock can be configured to engage one of the port 120 or port stem 122 in an interference fit, press-fit, snap-fit engagement. In an embodiment as shown in FIGS. 6A-6F, a cathlock lumen 142 can define a continuous or discontinuous, cylindrical or tapered shape, or combinations thereof. The cathlock lumen 142 can further include one or more abutments or stepped portions defining a change in lumen diameter.

With continued reference to FIGS. 1-2B, in an embodiment, a cathlock coupling tool 100 can be configured to engage the cathlock 140 and facilitate coupling the cathlock 140 with the catheter 130 and port stem 122, as described herein. In an embodiment, the tool 100 can be included as part of a kit including one of a port 120, port stem 122, catheter 90, a cathlock 140, or combinations thereof. In an embodiment, the tool 100 can generally include a body 102 extending longitudinally between a first end 114, proximate to the port 120, and a second end 116, proximate to the catheter 130. The body 102 can define a channel 103 extending longitudinally, along a central axis 90 between a first opening 110, proximate the port 120, and a second opening 112, proximate the catheter 130. The channel 103 can be configured to retain the cathlock 140 therein.

In an embodiment, an inner profile of the channel 103 can mirror an outer profile of the cathlock 140, as described herein. As such, the cathlock 140 can fit tightly within the channel 103 and the body 102 can retain the cathlock 140 therein. In an embodiment, the inner profile of the channel 103 can define a substantially cylindrical shape. In an embodiment, the inner profile of the channel 103 can define a tapered or frusto-conical shape. In an embodiment, the first opening 110 or the second opening 112 of the channel 103 can define a substantially circular cross-sectional shape. However, it will be appreciated that other cross-sectional shapes are contemplated including square, rectangular, hexagonal, elliptical, or the like.

In an embodiment a diameter *(d5)* of the first opening 110 can be greater than a diameter (*d6*) of the catheter-side opening 112. In an embodiment, the channel 103 can define a continuous change in diameter between the first opening 110 and the second opening 112. In an embodiment, the channel 103 can include a discontinuous change in diameter between the first opening 110 and the second opening 112. In an embodiment, a portion of the channel 103 can extend longitudinally at an angle of between 0°-90° relative to the longitudinal axis. In an embodiment, a portion of the channel 103 can extend longitudinally at an angle of between 1°-10° relative to the longitudinal axis. In an embodiment, the channel 103 can include an abutment surface configured to engage the cathlock 140 when the cathlock 140 is disposed within the channel 103. In an embodiment, the abutment surface can be a stepped portion, defining a change diameter of the channel 103 and configured to engage a stepped portion 148 of the cathlock 140. In an embodiment, the abutment can be a protrusion, or the like, configured to extend radially inwards from the surface of the channel 103 and engage a surface of the cathlock 140, as described in more detail herein.

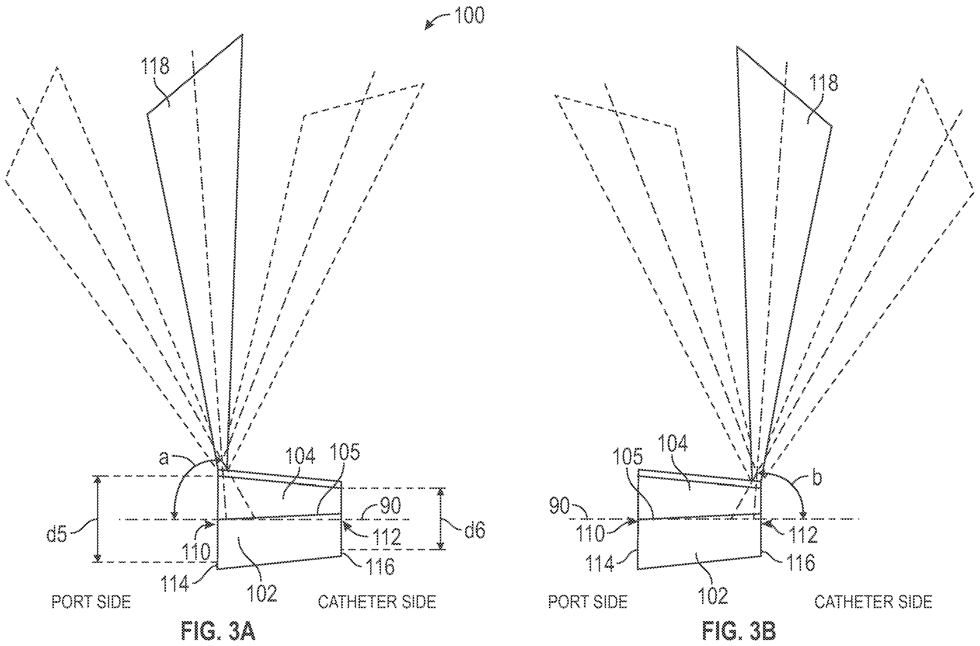

In an embodiment, as shown in FIGS. 3A-3B the tool 100 can further include a handle 118 extending from the body 102 and extending along an axis perpendicular to the central axis 90. In some embodiments, the handle 118 can define a concave outer profile, a convex outer profile, or combinations thereof to provide an ergonomic profile. In an embodiment, the handle 118 can include one or more finger grooves, abutments, finger pads, gripping features, or similar structures to facilitate grasping the handle 118. In an embodiment, the handle can include one or more materials with an increased friction co-efficient e.g. silicone rubber, or the like, to facilitate grasping the handle 118.

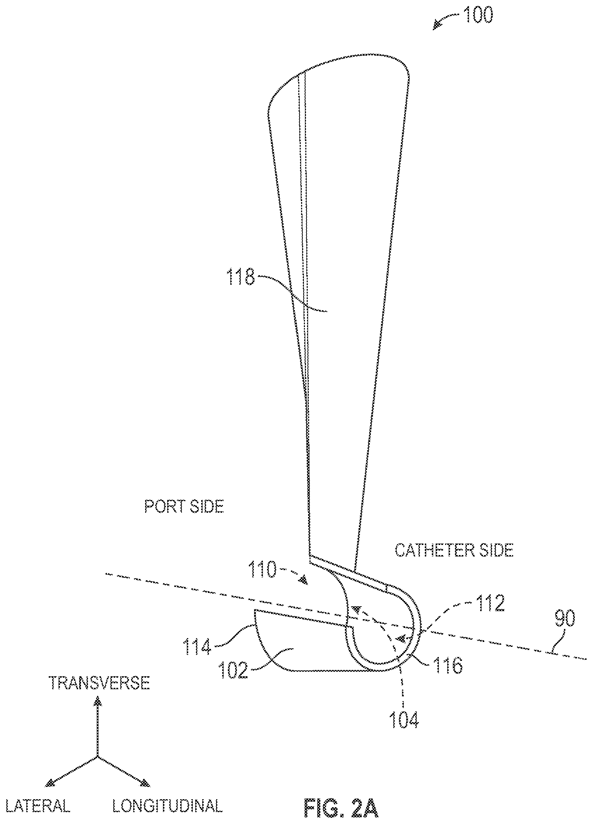

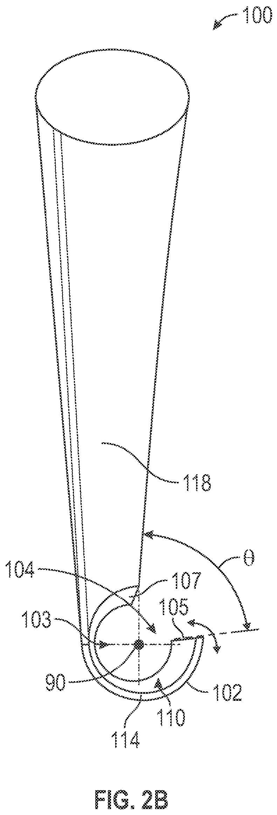

FIG. 2B illustrates an axial view of the cathlock coupling tool 100, viewed from a first end 114 of the body 102, i.e. from a port-side opening 110. In an embodiment, the body 102 can extend annularly about the central axis 90 to define the channel 103. In an embodiment, as shown in FIG. 2B, the body 102 can include an elongate opening 104 defined by a first edge 105 and a second edge 107, each extending longitudinally. The elongate opening 104 can communicate with the channel 103 and can be configured to allow ingress or egress of one of the catheter 130 or the cathlock 140 to or from the channel 103.

In an embodiment, the elongate opening 104 can extend annularly about the central axis 90 by an arc distance (*Θ*) of between 1° and 180°. In an embodiment, as shown in FIG. 2B, the elongate opening can be about 90°. In an embodiment, the elongate opening 104 can extend annularly about the central axis 90 by an arc distance (*Θ*) of 0°. Worded differently, the elongate opening 104 can define a slit such that the first edge 105 can contact the second edge 107.

In an embodiment, the body 102 can be formed of a resilient material and can elastically deform slightly. Exemplary materials can include plastic, polymer, metal, alloy, composite, or the like. In an embodiment, the first edge 105 of the elongate opening 104 can elastically deform relative to the second edge 107 to allow the elongate opening 104 to widen temporarily to allow ingress or egress of the catheter 130 or cathlock 140. Further, the elongate opening 104 be configured to allow the channel 103 to expand and receive the cathlock 140 therein. The body 102 can then grip and retain the cathlock 140 within the channel 103. In an embodiment, the elongate opening 104 can allow the body 102 to flex to receive different shapes and sizes of cathlock 140.

In an embodiment, the second edge 107 can be disposed adjacent the handle 118, as such, the portion of the body 102 adjacent the handle 118 and defining the second edge 107 can be form a wider wall thickness, and therefore define a less flexible, more rigid portion of the tool 100. By contrast, the first edge 105 is supported by a relatively thinner wall of the body 102 and as such can flexibly deform to move the first edge 105 relative to the second edge 107. As such, the cathlock 140 can be urged through the elongate opening 104 and the body 192 can engage the cathlock 140 in a snap-fit engagement. Advantageously, the snap-fit engagement can provide an audible or tactile alert to indicate to the clinician when the cathlock 140 is engaged with the tool 100. This can be important in the confined wetted environment of the tissue pocket where the body 102 and the cathlock 140 may not be directly observable.

In an embodiment, an edge of the elongate opening 104, e.g. the second edge 107 can be disposed adjacent the handle 118. Advantageously, the handle 118 disposed adjacent the elongate opening 104 can allow the clinician to guide the tool 100 relative to the cathlock 140. More specifically, the clinician can guide ingress or egress of the catheter 130 or the cathlock 140 through the elongate opening 104 by guiding the catheter 130/cathlock 140 along the handle 118 and into/out of the opening 104. This can be of particular importance when manipulating the body 102 of the tool 100 within the confines of the wetted environment of the tissue pocket where the body 102 and the cathlock 140 may not be directly observable.

In some embodiments, an outer surface of the body 102 can define a smooth or continuous outer profile. Advantageously, the smooth outer profile of the body 102 can mitigate trauma to the subcutaneous tissues as the tool body 102 is advanced axially or rotated about the central axis 90 to secure the cathlock 140 in place.

In an embodiment, as shown in FIG. 3A the handle 118 can be coupled to the body 102 at the first end 114 of the body 102. In an embodiment, the handle 118 can extend along an axis extending perpendicular to the central axis 90. In an embodiment, an axis of the handle 118 can align with a portion of the body adjacent the first end 114. In an embodiment, an axis of the handle 118 can align with the first end 114. In an embodiment, the handle 118 can extend along an axis extending at an angle (*a*) relative to the central axis 90. In an embodiment the angle (*a*) can be between 10° and 170°. In an embodiment the angle (*a*) can be between 50° and 130°. In an embodiment, the handle 118 can extend along an axis angled towards the first end, proximate the port stem 122, e.g. at an angle (*a*) of between 50° and 89°. Advantageously, the handle 118 coupled to the body 102 at the first end 114 can allow a clinician to "pull" the tool body 102 along the central axis 90 towards the port 120. The pulling action of the body 102 can allow the body 102 to maintain alignment with the central axis 90 as the body 102 is urged axially. In an embodiment, the handle 118 can be allowed to flex slightly relative to the body 102, to allow the body 192 to maintain alignment with central axis 90.

In an embodiment, as shown in FIG. 3B, the handle 118 can be coupled to the body 102 at the second end 116 of the body 102.. In an embodiment, an axis of the handle 118 can align with a portion of the body adjacent the second end 116. In an embodiment, an axis of the handle 118 can align with the second end 116. In an embodiment, the handle 118 can extend along an axis extending perpendicular to the central axis 90. In an embodiment, the handle 118 can extend along an axis extending at an angle (*b*) relative to the central axis 90.. In an embodiment the angle (*b*) can be between 10° and 170°. In an embodiment the angle (*b*) can be between 50° and 130°.

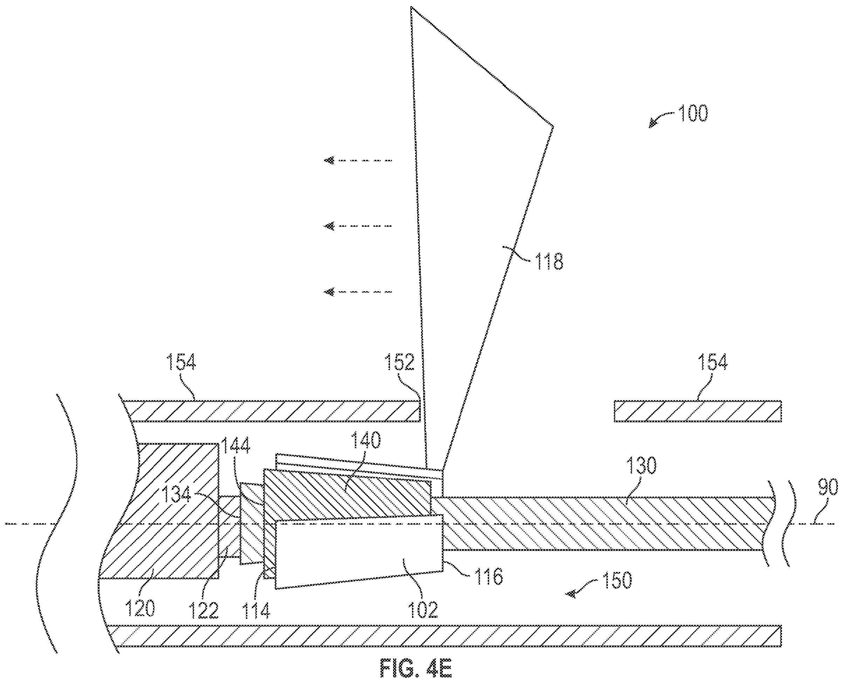

In an embodiment, the handle 118 extending from the second end 116 of the body 102 allows the body 102 to extend longitudinally from the handle axis. As such, the clinician can "push" the tool body 102 along the central axis 90 towards the port 120. Advantageously, as shown in FIG. 4E, the clinician can insert the body 102 underneath the skin surface 154, i.e. under the insertion site 152, to couple the cathlock 140 with the port 120 disposed within a tissue pocket, mitigating the handle 118 from impinging on the incision site 152 which might inhibit axial movement. In an embodiment, the handle 118 can be coupled to the body 102 at a point disposed between the first end 114 and the second end 116.

In an embodiment, an inner surface 106 of the channel 103 can include one or more abutments, for example one or more protrusions, abutments, ridges, or the like, extending radially inward towards the central axis 90 and configured to abut against a surface of the cathlock 140. In use, a cathlock 140 can be coupled with the tool body 102 and can be retained within the channel 103. In an embodiment, the cathlock 140 can slidably engage the channel 103 along the central axis 90 in a direction towards the second end 116, i.e. a "second direction." When the cathlock 140 is aligned with the channel 103, a surface of the cathlock 140 can abut against the abutment preventing further axial movement relative to the body 102 in the second direction. The clinician can manipulate the body 102, using the handle 118 to slide the cathlock 140 in the first direction, i.e. towards the port 120.

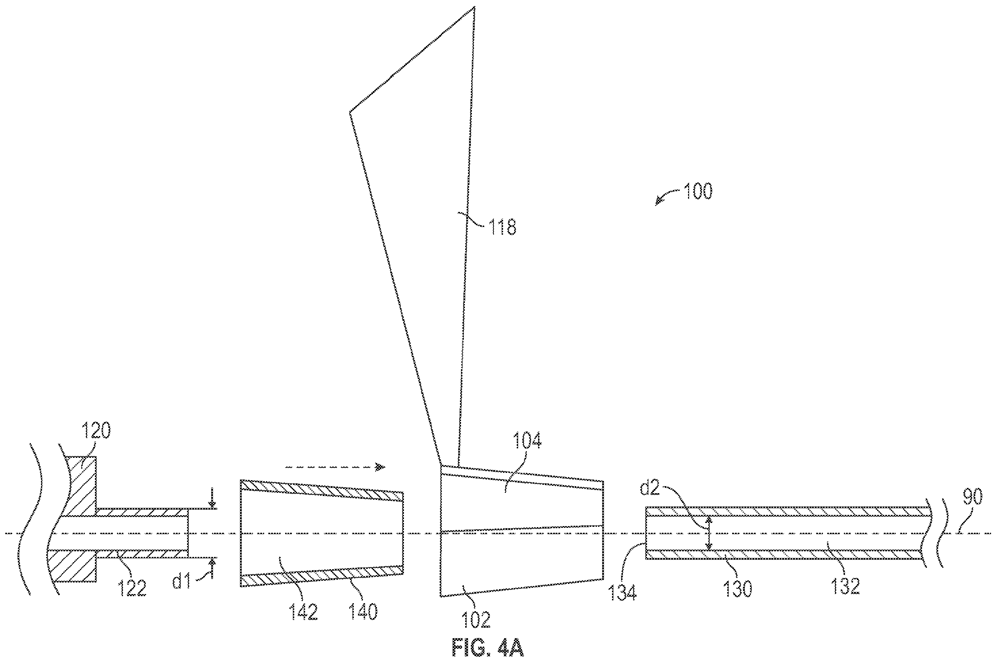

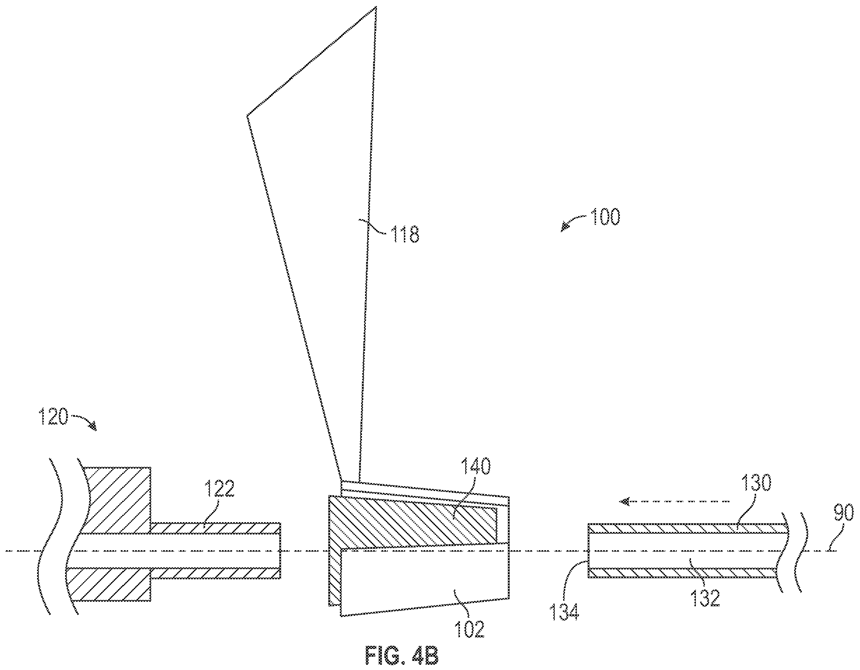

FIGS. 4A-4E show an exemplary method use for a cathlock coupling tool 100, configured to secure a cathlock 140 to a port 120/catheter 130 assembly, as described herein. As shown in FIG. 4A, the port stem 122, the catheter 130 and the cathlock 140 can align along the central axis 90. In an embodiment, as shown in FIGS. 4A-4B, the cathlock 140 can be retained within the channel 103 of the body 102 of the tool 100 before the cathlock 140 engages the catheter 130. The body 102 can be aligned with the axis 90 and the cathlock 140 can be slid axially in the second direction, i.e. towards the catheter 130, passing through the first opening 110, until the cathlock 140 is received within the channel 103 (FIG. 4A). The catheter 130 can then slidably engage the lumen 142 of the cathlock 140, along the central axis 90 (FIG. 4B).

In an embodiment, the cathlock 140 can be threaded onto the catheter 130 before engaging the tool 100. The catheter 130 can slidably engage the cathlock 140 through the cathlock lumen 142. The cathlock coupling tool 100 can then engage the catheter 130 at a point between the cathlock 140 and the distal tip of the catheter 130 by passing a portion of the catheter 130 through the elongate opening 104. The tool 100 can then be advanced axially in a first direction towards the cathlock 140 until the cathlock 140 is received and retained within the channel 103.

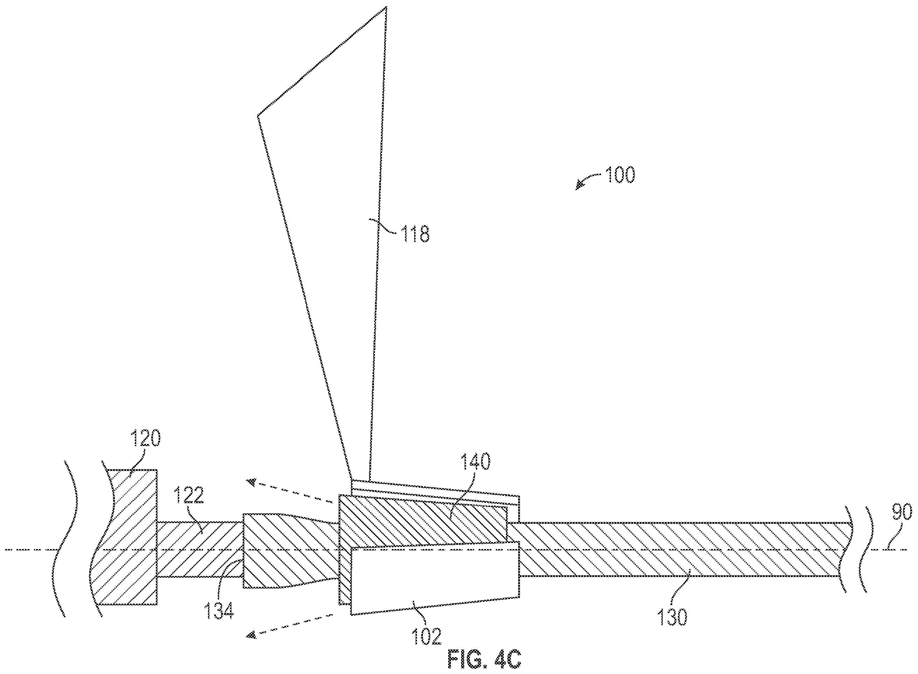

As shown in FIG. 4C, the catheter 130 can then be urged in the first direction to engage the port stem 122. The catheter lumen diameter (*d2*) can be less than an outer stem diameter (*d1*), as such the catheter 130 can be stretched over the port stem 122. In an embodiment, the port stem 122 can be formed of a substantially rigid material and the catheter can be formed of a compliant material. As the port stem 122 is urged into the lumen of the catheter 130, the catheter 130 can flexibly deform to engage the stem 122.

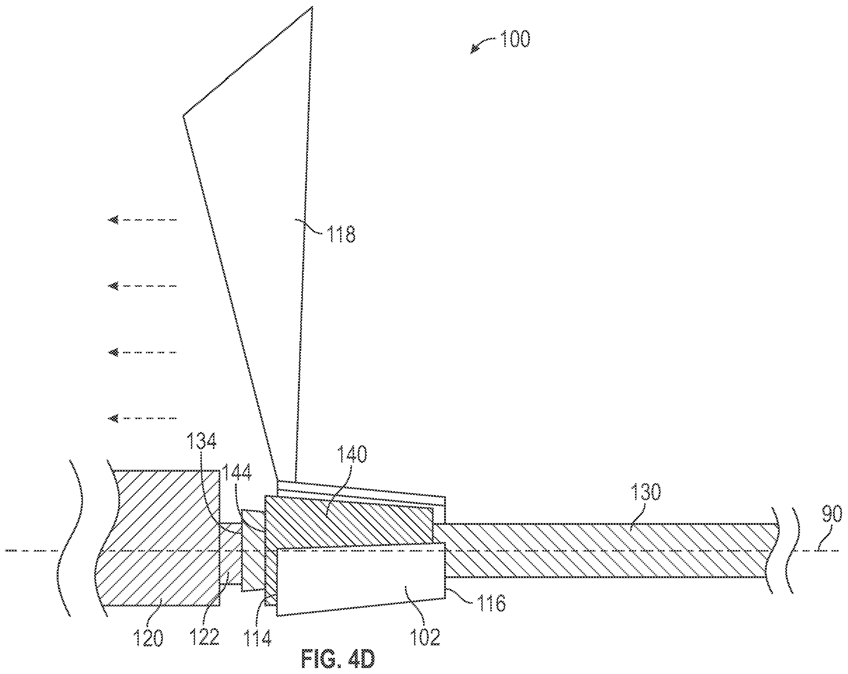

As shown in FIG. 4D, once the catheter 130 is coupled to the port stem 122, the cathlock 140 can be urged over the port stem 122. The cathlock 140 can be formed of a resilient material, and an inner surface of the cathlock 140 can engage an outer surface of the catheter 130 and can compress the catheter 130 on to the port stem 122. As shown in FIG. 4D, the cathlock coupling tool 100 can facilitate urging the cathlock 140 onto the port stem 122 / catheter 130 assembly.

With the cathlock 140 retained within the channel 103 of the body 102, an outer surface of the cathlock 140 can engage an inner surface of the channel 103 to prevent any further movement in the second direction, i.e. towards the catheter side. In an embodiment, the channel 103 can be tapered to prevent any further movement in the second direction. In an embodiment, the elongate opening 104 can allow the body 102 to flex perpendicular to the central axis 90, to grip the cathlock 140 in an interference fit. In an embodiment, the channel 103 can include one or more abutments extending radially inward and configured to engage a surface of the cathlock 140 to prevent any further movement in a second direction relative to the body 102.

With the cathlock 104 retained within the channel 103, a clinician can manipulate the body 102 using the handle 118. In an embodiment, as shown in FIG. 4D, the handle 118 can be coupled to the first end 114 of the body 102 and can pull the cathlock 140 onto the port stem 122.

In an embodiment, as shown in FIG. 4E, the handle 118 can be coupled to a second end 116 and can be configured to "push" the body 102 and the cathlock 140 disposed therein, onto the port stem 122. Advantageously, the body 102 can extend longitudinally from an axis of the handle 118 and can be urged into a tissue pocket 150. Advantageously, the handle 118 configured as such can mitigate contact between the handle 118 and an incision site 152, which may inhibit axial movement.

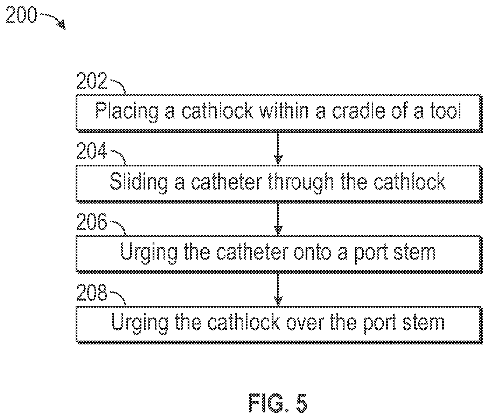

FIG. 5 illustrates a flow chart of the exemplary method 200 of coupling the cathlock 140 to the port 120 and the catheter assembly 130, in accordance with some embodiments. The method 200 includes placing the cathlock 140 within the channel 103 of the tool 100 (block 202). In some embodiments, placing includes sliding the cathlock 140 within the channel 103. The method 200 further includes sliding a catheter 130 through the cathlock 140 (block 204). The method 200 further includes urging the catheter 130 onto the port stem 122 (block 206). In some embodiments, urging the catheter 130 onto the port stem 122 includes a clinician manipulating one or more medical devices to urge the catheter 130 onto the port stem 122. In some embodiments, urging the catheter 130 onto the port stem 122 includes sliding the catheter 130 over the port stem 122. The method 200 further includes urging the cathlock 140 over the port stem 122 by manipulating the handle 118 of the cathlock coupling tool 100 (block 208). In some embodiments, manipulating the handle of the cathlock coupling tool 100 includes providing a pushing force on the handle 118 of the cathlock coupling tool 100 along the longitudinal axis. In some embodiments, manipulating the handle 118 of the cathlock coupling tool 100 includes providing a pulling force on the handle 118 of the cathlock coupling tool 100 along the longitudinal axis.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A system for coupling a cathlock (140) to a port stem (122) to secure a catheter (130) thereto, comprising:
a cathlock (140) defining a lumen extending longitudinally from a first end (144) to a second end (146); and
a tool (100) comprising:
a body (102) defining a channel (103) extending along a longitudinal axis (90) from a first end (114) to a second end (116), the channel including a first opening (110) at the first end (114) and configured to receive the cathlock (140) therein, the channel (103) further including a second opening (112) at the second end (116); and
a handle (118) extending from the body (102) at an angle relative to the longitudinal axis, the handle (118) coupled to one of the first end (114) or the second end of (116) the body (102),
wherein the body (102) further includes an elongate opening (104) defining a first edge (105) and a second edge (107), the elongate opening (104) extending longitudinally between the first end (114) and the second end (116), and communicating with the channel (103), and
**characterised in that** the body (102) is formed of a resilient material and elastically deformable.

2. The system according to claim 1, wherein the handle (118) is coupled to the first end (114) of the body (102) and the body (102) extends from the handle (118) to the second end (116).

3. The system according to claim 1, wherein the handle (118) is coupled to the second end (116) of the body (102) and the body (102) extends from the handle (118) to the first end (114).

4. The system according to any of claims 1-3, wherein the second edge (107) is disposed adjacent to the handle (118).

5. The system according to any one of claims 1-4, wherein the first edge (104) contacts the second edge (107) when the body (102) is in an unstressed state to form a slit.

6. The system according to any one of claims 1-4, wherein the elongate opening (104) extends through an arc distance between the first edge (105) and the second edge (107), the arc distance being between 0° and 90°.

7. The system according to any one of claims 1-6, wherein the channel (103) defines a cylindrical shape profile, optionally
wherein a wall of the channel (103) extends parallel to the longitudinal axis.

8. The system according to any one of claims 1-6, wherein the channel (103) defines a tapered profile, optionally
wherein a wall of the channel (103) extends at an angle relative to the longitudinal axis.

9. The system according to claim 8, wherein a diameter of the first opening (110) is larger than a diameter of the second opening (112), optionally
wherein the first opening (110) is larger than a diameter of the second end (146) of the cathlock (140), and the second opening (112) is smaller than the diameter of the second end (146) of the cathlock (140).

10. The system according to claim 9, wherein the channel (103) defines a continuous decrease in diameter between the first opening (110) and the second opening (112) or
wherein the channel (103) defines a discontinuous decrease in diameter between the first opening (110) and the second opening (112).

11. The system according to any one of claims 1-10, wherein the channel (103) includes an abutment configured to abut against a surface of the cathlock (140) and to inhibit further axial movement, and/or
wherein the inner profile of the channel (103) mirrors an outer profile of the cathlock (140).

12. A kit for accessing a vasculature of a patient, comprising:
the system of any of claims 1 to 11;
a port (120) having a port stem (122); and
a catheter (130) defining a catheter lumen (134) configured to engage the port stem (122),
wherein the cathlock (140) is configured to slidably engage the catheter (130), wherein the cathlock (140) is configured to engage an outer surface of the catheter (130) and to secure the catheter (130) to the port stem (122), and
wherein the tool (100) is configured to engage a surface of the cathlock (140).

13. The kit according to claim 12, wherein the port (122) includes a port body defining a reservoir (124) in fluid communication with a lumen of the port stem (122), optionally
wherein the port (120) includes a septum (126) disposed over the reservoir (124) and configured to provide percutaneous access thereto.

14. The kit according to any one of claims 12-13, wherein the cathlock (140) is configured to engage one of the port (120) or the port stem (122) in an interference fit, press-fit, or snap-fit engagement, and/or
wherein the cathlock (140) is configured to compress a portion of the catheter (130) onto the port stem (122).

15. The kit according to any one of claims 12-14, wherein the elongate opening (104) is configured to provide ingress or egress to of one of the cathlock (140) or the catheter (130) to or from the channel (103).

## Patentansprüche

1. System zum Koppeln eines Katheterverschlusses (140) mit einem Anschlussstutzen (122), um einen Katheter (130) daran zu sichern, umfassend:
einen Katheterverschluss (140), der ein Lumen definiert, das sich in Längsrichtung von einem ersten Ende (144) zu einem zweiten Ende (146) erstreckt; und
ein Werkzeug (100), umfassend:
einen Körper (102), der einen Kanal (103) definiert, der sich entlang einer Längsachse (90) von einem ersten Ende (114) zu einem zweiten Ende (116) erstreckt, wobei der Kanal eine erste Öffnung (110) an dem ersten Ende (114) einschließt und so konfiguriert ist, dass er den Katheterverschluss (140) darin aufnehmen kann, wobei der Kanal (103) weiter eine zweite Öffnung (112) an dem zweiten Ende (116) einschließt; und
einen Griff (118), der sich von dem Körper (102) in einem Winkel relativ zu der Längsachse erstreckt, wobei der Griff (118) mit einem von dem ersten Ende (114) oder dem zweiten Ende (116) des Körpers (102) gekoppelt ist,
wobei der Körper (102) weiter eine längliche Öffnung (104) einschließt, die eine erste Kante (105) und eine zweite Kante (107) definiert, wobei sich die längliche Öffnung (104) in Längsrichtung zwischen dem ersten Ende (114) und dem zweiten Ende (116) erstreckt und mit dem Kanal (103) in Verbindung steht, und **dadurch gekennzeichnet, dass** der Körper (102) aus einem elastischen Material gebildet und elastisch verformbar ist.

2. System nach Anspruch 1, wobei der Griff (118) mit dem ersten Ende (114) des Körpers (102) gekoppelt ist und sich der Körper (102) von dem Griff (118) zu dem zweiten Ende (116) erstreckt.

3. System nach Anspruch 1, wobei der Griff (118) mit dem zweiten Ende (116) des Körpers (102) gekoppelt ist und sich der Körper (102) von dem Griff (118) zu dem ersten Ende (114) erstreckt.

4. System nach einem der Ansprüche 1-3, wobei die zweite Kante (107) benachbart zu dem Griff (118) angeordnet ist.

5. System nach einem der Ansprüche 1-4, wobei die erste Kante (104) die zweite Kante (107) berührt, wenn sich der Körper (102) in einem unbelasteten Zustand befindet, um einen Schlitz zu bilden.

6. System nach einem der Ansprüche 1-4, wobei sich die längliche Öffnung (104) über einen Bogenabstand zwischen der ersten Kante (105) und der zweiten Kante (107) erstreckt, wobei der Bogenabstand zwischen 0° und 90° liegt.

7. System nach einem der Ansprüche 1-6, wobei der Kanal (103) ein zylindrisches Formprofil definiert, optional
wobei sich eine Wand des Kanals (103) parallel zu der Längsachse erstreckt.

8. System nach einem der Ansprüche 1-6, wobei der Kanal (103) ein sich verjüngendes Profil definiert, optional
wobei sich eine Wand des Kanals (103) in einem Winkel relativ zu der Längsachse erstreckt.

9. System nach Anspruch 8, wobei ein Durchmesser der ersten Öffnung (110) größer ist als ein Durchmesser der zweiten Öffnung (112), optional
wobei die erste Öffnung (110) größer ist als ein Durchmesser des zweiten Endes (146) des Katheterverschlusses (140) und die zweite Öffnung (112) kleiner ist als der Durchmesser des zweiten Endes (146) des Katheterverschlusses (140).

10. System nach Anspruch 9, wobei der Kanal (103) eine kontinuierliche Verringerung des Durchmessers zwischen der ersten Öffnung (110) und der zweiten Öffnung (112) definiert oder
wobei der Kanal (103) eine diskontinuierliche Verringerung des Durchmessers zwischen der ersten Öffnung (110) und der zweiten Öffnung (112) definiert.

11. System nach einem der Ansprüche 1-10, wobei der Kanal (103) ein Widerlager einschließt, das so konfiguriert ist, dass es an einer Oberfläche des Katheterverschlusses (140) anliegt und eine weitere axiale Bewegung verhindert, und/oder
wobei das Innenprofil des Kanals (103) ein Außenprofil des Katheterverschlusses (140) widerspiegelt.

12. Kit für den Zugang zu einem Gefäßsystem eines Patienten, umfassend:
das System nach einem der Ansprüche 1 bis 11;
einen Anschluss (120) mit einem Anschlussstutzen (122); und
einen Katheter (130), der ein Katheterlumen (134) definiert, das so konfiguriert ist, dass es mit dem Anschlussstutzen (122) in Eingriff kommt,
wobei der Katheterverschluss (140) so konfiguriert ist, dass er gleitend mit dem Katheter (130) in Eingriff kommt, wobei der Katheterverschluss (140) so konfiguriert ist, dass er mit einer Außenfläche des Katheters (130) in Eingriff kommt und den Katheter (130) an dem Anschlussstutzen (122) sichert, und
wobei das Werkzeug (100) so konfiguriert ist, dass es mit einer Oberfläche des Katheterverschlusses (140) in Eingriff kommt.

13. Kit nach Anspruch 12, wobei der Anschluss (122) einen Anschlusskörper einschließt, der ein Reservoir (124) definiert, das in Fluidverbindung mit einem Lumen des Anschlussstutzens (122) steht, optional
wobei der Anschluss (120) ein Septum (126) einschließt, das über dem Reservoir (124) angeordnet und so konfiguriert ist, dass es einen perkutanen Zugang dazu bereitstellt.

14. Kit nach einem der Ansprüche 12-13, wobei der Katheterverschluss (140) so konfiguriert ist, dass er mit einem von dem Anschluss (120) oder dem Anschlussstutzen (122) in einer Presspassung, einer Einpresspassung oder einem Schnappverbindungseingriff in Eingriff kommt, und/oder
wobei der Katheterverschluss (140) so konfiguriert ist, dass er einen Abschnitt des Katheters (130) auf den Anschlussstutzen (122) drückt.

15. Kit nach einem der Ansprüche 12-14, wobei die längliche Öffnung (104) so konfiguriert ist, dass sie einen Eingang oder Ausgang für entweder den Katheterverschluss (140) oder den Katheter (130) zu oder von dem Kanal (103) bereitstellt.

## Revendications

1. Système de couplage d'un verrou de cathéter (140) à une tige d'orifice (122) pour y fixer un cathéter (130), comprenant :
un verrou de cathéter (140) définissant une lumière s'étendant longitudinalement d'une première extrémité (144) jusqu'à une seconde extrémité (146) ; et
un outil (100) comprenant :
un corps (102) définissant un canal (103) s'étendant le long d'un axe longitudinal (90) depuis une première extrémité (114) jusqu'à une seconde extrémité (116), le canal incluant une première ouverture (110) au niveau de la première extrémité (114) et configuré pour recevoir le verrou de cathéter (140) à l'intérieur, le canal (103) incluant en outre une seconde ouverture (112) à la seconde extrémité (116) ; et
une poignée (118) s'étendant à partir du corps (102) selon un angle par rapport à l'axe longitudinal, la poignée (118) étant couplée à l'une de la première extrémité (114) ou de la seconde extrémité (116) du corps (102),
dans lequel le corps (102) inclut en outre une ouverture allongée (104) définissant un premier bord (105) et un second bord (107), l'ouverture allongée (104) s'étendant longitudinalement entre la première extrémité (114) et la seconde extrémité (116) et communiquant avec le canal (103), et
**caractérisé en ce que**
le corps (102) est formé d'un matériau résilient et est déformable élastiquement.

2. Système selon la revendication 1, dans lequel la poignée (118) est couplée à la première extrémité (114) du corps (102) et le corps (102) s'étend de la poignée (118) à la seconde extrémité (116).

3. Système selon la revendication 1, dans lequel la poignée (118) est couplée à la seconde extrémité (116) du corps (102) et le corps (102) s'étend depuis la poignée (118) jusqu'à la première extrémité (114).

4. Système selon l'une quelconque des revendications 1 à 3, dans lequel le second bord (107) est disposé de façon adjacente à la poignée (118).

5. Système selon l'une quelconque des revendications 1 à 4, dans lequel le premier bord (104) entre en contact avec le second bord (107) lorsque le corps (102) est dans un état non contraint pour former une fente.

6. Système selon l'une quelconque des revendications 1 à 4, dans lequel l'ouverture allongée (104) s'étend sur une distance d'arc entre le premier bord (105) et le second bord (107), la distance d'arc étant comprise entre 0° et 90°.

7. Système selon l'une quelconque des revendications 1 à 6, dans lequel le canal (103) définit un profil de forme cylindrique, éventuellement
dans lequel une paroi du canal (103) s'étend parallèlement à l'axe longitudinal.

8. Système selon l'une quelconque des revendications 1 à 6, dans lequel le canal (103) définit un profil effilé, optionnellement
dans lequel une paroi du canal (103) s'étend selon un angle par rapport à l'axe longitudinal.

9. Système selon la revendication 8, dans lequel le diamètre de la première ouverture (110) est supérieur au diamètre de la seconde ouverture (112), optionnellement
dans lequel la première ouverture (110) est plus grande qu'un diamètre de la seconde extrémité (146) du verrou de cathéter (140), et la seconde ouverture (112) est plus petite que le diamètre de la seconde extrémité (146) du verrou de cathéter (140).

10. Système selon la revendication 9, dans lequel le canal (103) définit une diminution continue du diamètre entre la première ouverture (110) et la seconde ouverture (112) ou
dans lequel le canal (103) définit une diminution discontinue du diamètre entre la première ouverture (110) et la seconde ouverture (112).

11. Système selon l'une quelconque des revendications 1 à 10, dans lequel le canal (103) inclut une butée configurée pour venir en butée contre une surface du verrou de cathéter (140) et pour empêcher tout mouvement axial supplémentaire, et/ou
dans lequel le profil intérieur du canal (103) reflète un profil extérieur du verrou de cathéter (140).

12. Kit d'accès au système vasculaire d'un patient, comprenant :
le système selon l'une quelconque des revendications 1 à 11 ;
un orifice (120) présentant une tige d'orifice (122) ; et
un cathéter (130) définissant une lumière de cathéter (134) configurée pour s'engager avec la tige d'orifice (122),
dans lequel le verrou de cathéter (140) est configuré pour s'engager de manière coulissante avec le cathéter (130), dans lequel le verrou de cathéter (140) est configuré pour s'engager avec une surface extérieure du cathéter (130) et pour fixer le cathéter (130) à la tige d'orifice (122), et
dans lequel l'outil (100) est configuré pour s'engager avec une surface du verrou de cathéter (140).

13. Kit selon la revendication 12, dans lequel l'orifice (122) inclut un corps d'orifice définissant un réservoir (124) en communication fluidique avec une lumière de la tige d'orifice (122), éventuellement
dans lequel l'orifice (120) inclut un septum (126) disposé sur le réservoir (124) et configuré pour fournir un accès percutané à celui-ci.

14. Kit selon l'une quelconque des revendications 12 à 13, dans lequel le verrou de cathéter (140) est configuré pour s'engager avec l'un de l'orifice (120) ou de la tige d'orifice (122) dans un ajustement par interférence, par pression ou par encliquetage, et/ou
dans lequel le verrou de cathéter (140) est configuré pour comprimer une partie du cathéter (130) sur la tige d'orifice (122).

15. Kit selon l'une quelconque des revendications 12 à 14, dans lequel l'ouverture allongée (104) est configurée pour permettre l'entrée ou la sortie de l'un parmi le verrou de cathéter (140) ou le cathéter (130) vers ou depuis le canal (103).
